# EUROPEAN PATENT APPLICATION

(11) **EP 4 755 345 A1**
(43) Date of publication of application: **10.06.2026**
(21) Application number: 24921153.3
(22) Date of filing: 22.03.2024
(51) Int. Cl.: A61F 2/04

(54) **GRADABLE DETACHABLE DIGESTIVE TRACT IMPLANT CANNULA AND DIGESTIVE TRACT IMPLANT KIT**

(30) Priority: 29.01.2024 CN 202410126018
(71) Applicant: Hangzhou Tangji Medical Technology Co., Ltd., Hangzhou, Zhejiang 310052 (CN)
(72) Inventor: LI, Wenyu, Hangzhou, Zhejiang 310052 (CN)
(74) Representative: Vogelbruch, Keang
(86) International application number: PCT/CN2024/083188
(87) International publication number: WO 2025/161108

(57) **Abstract**

The present application relates to the field of medical instruments. Disclosed are a gradable detachable digestive tract implant cannula and a digestive tract implant kit. The gradable detachable digestive tract implant cannula has a cannula length of 60-150 cm and has a proximal end and a distal end. At least one low-stress portion is arranged on the cannula at a distance of 30-120 cm from the proximal end. When the cannula becomes obstructed, the low-stress portion can undergo fracture in response to intestinal peristaltic forces. The digestive tract implant kit comprises a scaffold and the gradable detachable digestive tract implant cannula, and the proximal end of the gradable detachable digestive tract implant cannula is connected to the scaffold. When the cannula provided by the present application becomes obstructed, a rear end portion of the cannula can automatically undergo graded detachment in response to intestinal peristaltic forces, thereby avoiding intestinal obstruction caused by the obstruction of the cannula and thus significantly improving the safety of the instrument implanted in the body.

## Description

### CROSS-REFERENCE TO THE RELATED APPLICATIONS

This application claims priority to Chinese Patent Application No. 202410126018.X filed with the China National Intellectual Property Administration on January 29, 2024, entitled "GRADABLE DETACHABLE DIGESTIVE TRACT IMPLANT SLEEVE AND DIGESTIVE TRACT IMPLANT KIT," the entire contents of which are incorporated herein by reference.

### TECHNICAL FIELD

The present application relates to the field of medical devices, specifically to a gradable detachable digestive tract implant sleeve (cannula) and a digestive tract implant kit.

### BACKGROUND ART

The global annual expenditure attributable to obesity has reached a staggering 10 trillion RMB, accounting for approximately 2.8% of the gross world product (GWP). In the United States, the costs for treating obesity-related diseases account for nearly 17% of total healthcare expenditures. According to obesity data published by *The Lancet* in 2021: the overweight rate among adults aged 18 and above was 34.3%, the obesity rate was 16.4%, totaling 50.7%; the number of obese individuals in China exceeded 200 million, while the global obese population surpassed 700 million. Meanwhile, 80-90% of obese patients suffer from type 2 diabetes. According to data released by the International Diabetes Federation (IDF) in December 2021, 537 million adults aged 20-79 were living with diabetes, accounting for 10.5% of the global population in this age group. The number is projected to rise to 643 million (11.3%) by 2030 and 783 million (12.2%) by 2045. Statistics from the IDF and numerous domestic studies show that type 2 diabetes is the most prevalent form of diabetes, accounting for approximately 90% of all diabetes cases worldwide. Meanwhile, the global healthcare expenditure caused by diabetes has grown considerably. Healthcare spending for adults aged 20-79 surged from 232 billion USD in 2007 to 966 billion USD in 2021. The future market size is expected to reach trillion-dollar level. Diabetes has become the third major category of non-communicable diseases following cardiovascular diseases and cancer. With the number of patients increasing year by year, it has evolved into a global public health issue threatening human health. Thus, there is an urgent need for a medical technology to address the aforementioned challenges.

Currently, the pathogenesis of type 2 diabetes remains not fully elucidated. Despite existing oral medications and insulin therapies, many patients still experience poor glycemic control and these treatments cannot effectively prevent the onset or progression of complications. It can be stated that there are currently no effective curative therapeutic approaches available. Bariatric surgery yields relatively favorable outcomes for the aforementioned diseases, but it involves irreversible physiological trauma and carries certain risks of mortality and postoperative complications, such as gastrointestinal leakage, anastomotic stricture, and dumping syndrome. Since 2008, US-based GI Dynamics Inc. has developed the duodenal-jejunal bypass liner (DJBL) based on the principles of Roux-en-Y gastric bypass (RYGB, a type of metabolic surgery). By placing a sleeve in the duodenum and proximal jejunum, it isolates chyme from contact with the intestinal wall. After ingested nutrients enter the stomach, they pass through the sleeve into the proximal jejunum. Pancreatic juice and bile are secreted naturally, flow downward between the sleeve and the intestinal wall, and mix with chyme at the distal end of the DJBL (i.e., in the jejunum). This reduces nutrient absorption, thereby achieving the therapeutic goal of treating obesity and type 2 diabetes.

A study by Forner et al. found that the Endobarrier developed by GI Dynamics Inc. had a 5% incidence of device obstruction after implantation in the human body [Obes Surg 27(12): 3306-3313.]. Internal device obstruction may necessitate premature device explantation in mild cases, while severe cases can lead to intestinal obstruction or, in extreme cases, intestinal intussusception. Intestinal intussusception typically requires immediate surgical intervention; otherwise, it may be life-threatening or even fatal. Therefore, it is crucial to reduce the incidence of such device obstructions and improve the safety of implanted devices.

In view of the above, the present application is hereby proposed.

### SUMMARY

The objectives of the present application include, for example, providing a gradable detachable digestive tract implant sleeve and a digestive tract implant kit, aiming to ameliorate at least one of the problems mentioned in the background art.

The embodiments of the present application can be implemented as follows.

In the first aspect, the present application provides a gradable detachable digestive tract implant sleeve, where the sleeve has a length of 60-150 cm, and the sleeve includes a proximal end and a distal end, with at least one low-stress region (portion) provided on the sleeve at a distance of 30-120 cm from the proximal end, where
the low-stress region is capable of, in response to intestinal peristaltic force, fracturing upon occurrence of an obstruction, thereby causing automatic detachment of a segment of the sleeve from a fractured site to the distal end.

In an optional embodiment, a fracture stress of the low-stress region is 1-12 N.

In an optional embodiment, there are a plurality of low-stress regions, and the plurality of low-stress regions are evenly distributed along a length direction of the sleeve.

In an optional embodiment, stresses of the plurality of low-stress regions are designed in a gradient pattern; and the fracture stresses of the plurality of low-stress regions decrease gradually along the length direction of the sleeve.

In an optional embodiment, a stress gradient of the low-stress regions designed in the gradient pattern is at least 1 N.

In an optional embodiment, the sleeve is made of at least one material selected from the group consisting of PTFE, FEP, ePTFE, PU, LDPE, LLDPE, and PE.

In an optional embodiment, a wall thickness of the sleeve is 0.01-0.05 mm.

In an optional embodiment, each low-stress region includes a body which is a part of the sleeve, and a frangible perforated line provided circumferentially around the body, where the frangible perforated line is composed of a plurality of spaced-apart frangible through holes.

In an optional embodiment, the frangible perforated line can be formed by laser etching, stamping, perforated-line cutter cutting or oscillating-knife cutting.

In the second aspect, the present application provides a digestive tract implant kit, including a stent (scaffold) and the gradable detachable digestive tract implant sleeve according to any one of the aforementioned embodiments, with the proximal end of the gradable detachable digestive tract implant sleeve connected to the stent.

The beneficial effects of the embodiments of the present application include, for example:

For the gradable detachable digestive tract implant sleeve provided in the present application, when device obstruction occurs, due to the configuration of the low-stress region, the low-stress region can, in response to intestinal peristaltic force, fracture upon occurrence of device obstruction, thereby causing detachment of a segment of the sleeve. This can prevent the occurrence of intestinal obstruction induced by device obstruction, thus significantly improving the safety of the device implanted in the body.

### BRIEF DESCRIPTION OF THE DRAWINGS

To illustrate the technical solutions of the embodiments of the present application more clearly, the drawings required for the embodiments will be briefly described below. It should be understood that the following drawings only show certain embodiments of the present application and therefore should not be regarded as limiting the scope. For those of ordinary skill in the art, other relevant drawings could be obtained based on these drawings without creative efforts.
FIG. 1 is a schematic structural diagram showing the cooperation between a sleeve and a stent provided in an embodiment of the present application; and
FIG. 2 is a schematic diagram of simulated intestinal obstruction occurrence in an experimental example.

Reference Numerals: 100-sleeve; 101-proximal end; 102-distal end; 110-low-stress region; 111-frangible perforated line; 200-stent; 300-simulator; 301-adjustable valve; 302-placement opening.

### DETAILED DESCRIPTION OF THE EMBODIMENTS

To clarify the objectives, technical solutions, and advantages of the embodiments of the present application, the technical solutions in the embodiments of the present application will be clearly and completely described below in conjunction with the drawings in the embodiments of the present application. It is evident that only some but not all of the embodiments of the present application are described. Generally, the components of the embodiments of the present application described and illustrated in the drawings herein may be arranged and designed in various different configurations.

Therefore, the following detailed description of the embodiments of the present application provided in the drawings is not intended to limit the scope of the claimed present application, but merely represents the selected embodiments of the present application. Based on the embodiments in the present application, all other embodiments obtained by those of ordinary skill in the art without creative efforts shall fall within the scope of protection of the present application.

It should be noted that similar reference numerals and letters denote similar items in the following drawings, and thus, once an item is defined in one drawing, it does not need to be further defined or explained in subsequent drawings.

In the description of the present application, it should be noted that the terms "upper," "lower," "inner," "outer," and the like, if appear, indicate orientation or positional relationships based on the orientation or positional relationships shown in the drawings, or the orientation or positional relationships commonly placed when the product of this application is used, which are merely for the convenience of describing the present application and simplifying the description, rather than indicating or implying that the referred device or element must have a specific orientation or be constructed and operated in a specific orientation, and thus cannot be construed as a limitation to the present application.

In addition, the terms "first," "second" and the like, if appear, are only used for distinguishing descriptions, and cannot be understood as indicating or implying relative importance.

It should be noted that the features of the embodiments of the present application may be combined with each other provided that no conflict arises.

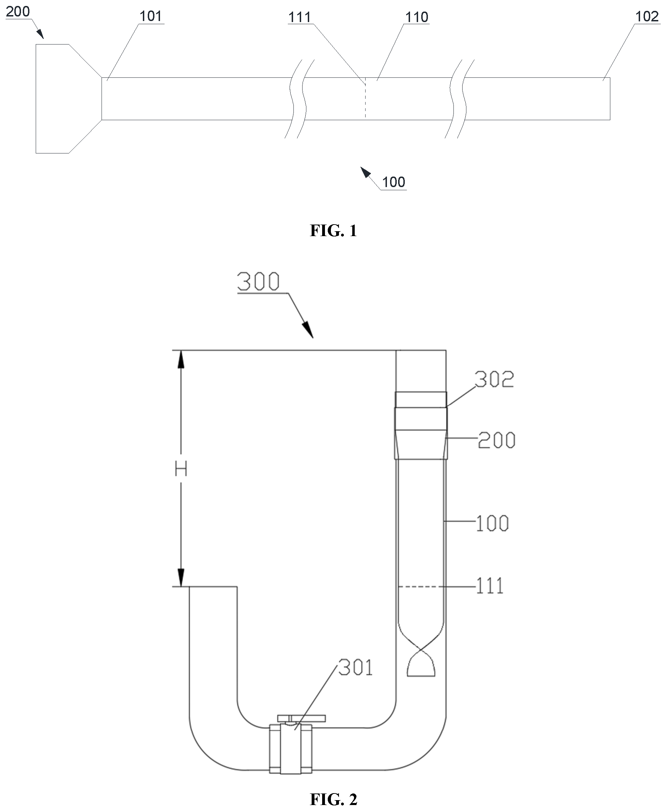

Referring to FIG. 1, the present embodiment of the present application provides a gradable detachable digestive tract implant sleeve 100. The sleeve 100 has a length of 60-150 cm. The sleeve 100 includes a proximal end 101 and a distal end 102. At least one low-stress region 110 is provided on the sleeve 100 at a distance from the proximal end 101.

The low-stress region 110 is capable of, in response to intestinal peristaltic force, fracturing upon occurrence of an obstruction, thereby causing automatic detachment of a segment of the sleeve 100 from a fractured site to the distal end 102.

For the gradable detachable digestive tract implant sleeve 100 provided in the present application, when device obstruction occurs, the specifically configured low-stress region 110 is capable of fracturing under stress in response to intestinal peristaltic force, thereby causing automatic detachment of this segment of the sleeve 100 from the fractured site to the distal end 102. This can prevent intestinal obstruction caused by device obstruction, thereby significantly improving the safety of the device implanted in the body.

The proximal end 101 refers to the end close to the duodenal bulb during implantation, while the distal end 102 refers to the end away from the duodenal bulb.

Optionally, the fracture stress of the low-stress region 110 is 1-12 N.

When the fracture stress of the responsive low-stress region 110 falls within the range of 1-12 N, the low-stress region 110 can fracture under the action of intestinal peristaltic force in the event of device obstruction.

Preferably, to fully prevent the occurrence of intestinal obstruction in case of device obstruction, a plurality of low-stress regions 110 are provided, and the plurality of low-stress regions 110 are evenly distributed along the length direction of the sleeve 100.

Further, the fracture stresses of the plurality of low-stress regions 110 decrease gradually along the length direction of the sleeve 100, meaning that the regions located farther back are more prone to fracture.

Specifically, for example, the stresses of the plurality of low-stress regions 110 are designed in a gradient pattern; and the fracture stresses decrease gradually along the length direction of the sleeve 100.

Further, a stress gradient of the low-stress regions designed in the gradient pattern is at least 1 N. For instance, 2, 3 or 4 low-stress regions 110 may be provided, where the fracture stress of the previous region is 1 N, 2 N or 3 N greater than that of the subsequent one.

Specifically, the sleeve 100 is made of at least one material selected from the group consisting of polytetrafluoroethylene (PTFE), fluorinated ethylene propylene (FEP) copolymer, expanded polytetrafluoroethylene (ePTFE), polyurethane (PU), low-density polyethylene (LDPE), and linear low-density polyethylene (LLDPE).

Further, each low-stress region 110 includes a body that is part of the sleeve 100, and a frangible perforated line 111 provided circumferentially around the body, where the frangible perforated line 111 is composed of a plurality of spaced-apart frangible through holes.

The frangible perforated line 111 is a practical implementation form of the low-stress region 110 that facilitates fracture stress regulation. Adjusting the size of the frangible through holes or the spacing between the frangible through holes enables to achieve different fracture stress levels; for example, larger frangible through holes or greater spacing between the frangible through holes results in a lower fracture stress, and vice versa.

It should be noted that although frangible through holes are provided, since chyme is a high-viscosity fluid with large particles, there is no need to worry about leakage of chyme as long as the aperture of the through holes is not excessively large.

Further, to ensure the operational strength of the sleeve 100, a wall thickness of the sleeve 100 at the frangible through holes is 0.01-0.05 mm.

Further, the frangible perforated line 111 can be formed by laser etching, stamping, perforated-line cutter cutting or oscillating-knife cutting.

Embodiments of the present application also provide a digestive tract implant kit, which includes a stent 200 and the gradable detachable digestive tract implant sleeve 100 provided in the embodiments of the present application, with the proximal end 101 of the gradable detachable digestive tract implant sleeve 100 connected to the stent 200.

### Example 1

The sleeve 100 provided in the present example has a total length of 60 cm. A frangible perforated line 111 is circumferentially provided at a distance of 40 cm from the proximal end 101, with the fracture stress of the frangible perforated line 111 being approximately 4 N. The entire sleeve 100 is made of an LDPE material, and the sleeve 100 has a wall thickness of approximately 0.02 mm. The low-stress region 110 is fabricated by perforated-line cutter cutting.

### Example 2

The sleeve 100 provided in the present example has a total length of 100 cm. The frangible perforated line 111 is circumferentially provided respectively at positions 80 cm and 90 cm from the proximal end 101. From the proximal end 101 to the distal end 102, the fracture stresses of the two frangible perforated lines 111 are approximately 7 N and 4 N respectively. The entire sleeve 100 is made of an LDPE/LDPE composite material, and the sleeve 100 has a wall thickness of approximately 0.02 mm. The low-stress region 110 is fabricated by stamping.

### Example 3

The sleeve 100 provided in the present example has a total length of 150 cm. The frangible perforated line 111 is circumferentially provided respectively at positions 90 cm, 120 cm, and 140 cm from the proximal end 101. From the proximal end 101 to the distal end 102, the fracture stresses of the three frangible perforated lines 111 are approximately 12 N, 8 N, and 3 N respectively. The entire sleeve 100 is made of an ePTFE/FEP composite material, and the sleeve 100 has a wall thickness of approximately 0.01 mm. The low-stress region 110 is fabricated by laser etching.

### Example 4

The sleeve 100 provided in the present example has a total length of 60 cm. The frangible perforated line 111 is circumferentially provided respectively at positions 40 cm and 50 cm from the proximal end 101. The fracture stresses of the frangible perforated lines 111 are approximately 8 N and 3 N respectively. The entire sleeve 100 is made of a PU material, and the sleeve 100 has a wall thickness of approximately 0.02 mm. The low-stress region 110 is fabricated by oscillating-knife cutting.

### Comparative Example

The present comparative example is substantially the same as Example 1, with the only difference being that no low-stress region 110 is provided.

### Experimental Example

Upon obstruction, the sleeve 100 will be subjected to pulling forces from duodenal peristalsis. According to the study by Gregerson et al., the pressure of intestinal peristalsis can reach up to 80 mmHg (108.8 cmH₂O) (Gregerson, H., et al. Essentials of Experimental Surgery: Gastroenterology. CRC Press, 1996). When the sleeve 100 is blocked, the low-stress region fractures under the pulling force of duodenal peristalsis. After fracture, the distal end 102 of the sleeve 100 is expelled from the body with intestinal peristalsis. Meanwhile, the low-stress region 110 shall be capable of withstanding the normal intestinal peristaltic force when no sleeve obstruction occurs. Based on this principle, an intestinal obstruction simulator was designed to simulate the detachment effect of the gradable detachable digestive tract sleeve 100 provided in the present application in the event of obstruction of the digestive tract sleeve 100, as shown in FIG. 2. The simulator 300 is capable of providing a certain pressure difference H, and is equipped with an adjustable valve 301 and a placement opening 302 for accommodating the digestive tract implant kit provided in the present application.

As shown in FIG. 2, the adjustable valve 301 was closed and water was injected into the device until the water level reaches approximately 2 cm below the placement opening 302. Then, the membrane tube of the sleeve 100 was tightly tied off with a thread to simulate an obstruction. The digestive tract kits respectively composed of the gradable detachable digestive tract sleeve 100 provided in each of Examples 1-4 and the sleeve provided in the Comparative Example were each placed into the placement opening 302. Water was then gradually injected into the sleeve 100 (note: it is necessary to ensure that the exterior of the sleeve was completely filled with water before injecting water into the sleeve 100). The pressure difference was adjusted to 100 cmH₂O by injecting different volumes of water, after which the adjustable valve 301 was opened and the pressure difference at which the low-stress region 110 fractures was recorded. For sleeves 100 with multiple low-stress regions 110, the region where fracture occurred was recorded.

The experimental results are presented in Table 1.

**Table 1 Fracture Record of Each Example and Comparative Example**

| Group | Whether fracture occurs at 100 cmH₂O with distal end not tied off | Whether fracture occurs at 100 cmH₂O with distal end tied off (simulated obstruction) | Note |
|---|---|---|---|
| Example 1 | No | Yes | / |
| Example 2 | No | Yes | Fracture at 90 cm |
| Example 3 | No | Yes | Fracture at 140 cm |
| Example 4 | No | Yes | Fracture at 50 cm |
| Comparative Example | No | Yes | / |

As can be seen from Table 1, the sleeves 100 provided in each example of the present application all detached under the simulated device obstruction environment of the simulator 300, indicating that these sleeves 100 can effectively prevent intestinal obstruction caused by device obstruction after being implanted into the intestinal tract, thereby significantly improving the safety of the device implanted in the body.

In contrast, as no low-stress region 110 was provided in the Comparative Example, no fracture or detachment occurred, which indicates that the configured low-stress region 110 should have a relatively low fracture stress; otherwise, it would be difficult to achieve the fracture and detachment of the sleeve 100.

In summary, for the gradable detachable digestive tract implant sleeve 100 provided in the examples of the present application, when device obstruction occurs, due to the configuration of the low-stress region 110, the low-stress region 110 is capable of fracturing in response to intestinal peristaltic force, causing detachment of a rear segment of the sleeve 100. This can prevent intestinal obstruction caused by device obstruction, thus greatly enhancing the safety of the device implanted in the body.

The above are merely for specific embodiments of the present application, but the scope of protection of the present application is not limited thereto. Any variation or replacement readily conceivable by those skilled in the art within the technical scope disclosed in the present application shall fall within the scope of protection of the present application. Therefore, the scope of protection of the present application shall be subject to the scope of protection defined by the claims.

### INDUSTRIAL APPLICABILITY

The gradable detachable digestive tract implant sleeve provided in the present application is configured with the low-stress region. When device obstruction occurs, the low-stress region is capable of fracturing in response to intestinal peristaltic force, causing detachment of the rear segment of the sleeve. This can prevent intestinal obstruction caused by device obstruction, thereby significantly improving the safety of the device implanted in the body.

## Claims

1. A gradable detachable digestive tract implant sleeve, **characterized in that** the sleeve has a length of 60-150 cm, the sleeve comprises a proximal end and a distal end, and at least one low-stress region is provided on the sleeve at a distance of 30-120 cm from the proximal end, wherein
the low-stress region is configured to fracture in response to intestinal peristaltic force upon occurrence of an obstruction, allowing automatic detachment of a segment of the sleeve from a fractured site to the distal end.

2. The gradable detachable digestive tract implant sleeve according to claim 1, **characterized in that** a fracture stress of the low-stress region is 1-12 N.

3. The gradable detachable digestive tract implant sleeve according to claim 2, **characterized in that** there are a plurality of low-stress regions, and the plurality of low-stress regions are evenly distributed along a length direction of the sleeve.

4. The gradable detachable digestive tract implant sleeve according to claim 3, **characterized in that** stresses of the plurality of low-stress regions are designed in a gradient pattern; and fracture stresses of the plurality of low-stress regions decrease gradually along the length direction of the sleeve.

5. The gradable detachable digestive tract implant sleeve according to claim 4, **characterized in that** a stress gradient of the low-stress regions designed in the gradient pattern is at least 1 N.

6. The gradable detachable digestive tract implant sleeve according to any one of claims 1 to 5, **characterized in that** the sleeve is made of at least one material selected from the group consisting of PTFE, FEP, ePTFE, PU, LDPE, LLDPE, and PE.

7. The gradable detachable digestive tract implant sleeve according to any one of claims 1 to 6, **characterized in that** a wall thickness of the sleeve is 0.01-0.05 mm.

8. The gradable detachable digestive tract implant sleeve according to any one of claims 1 to 7, **characterized in that** each low-stress region comprises a body which is a part of the sleeve, and a frangible perforated line circumferentially provided around the body, wherein the frangible perforated line is composed of a plurality of spaced-apart frangible through holes.

9. The gradable detachable digestive tract implant sleeve according to claim 8, **characterized in that** the frangible perforated line is formed by laser etching, stamping, perforated-line cutter cutting or oscillating-knife cutting.

10. A digestive tract implant kit, **characterized by** comprising a stent and the gradable detachable digestive tract implant sleeve according to any one of claims 1 to 9, wherein the proximal end of the gradable detachable digestive tract implant sleeve is connected to the stent.
